## Europäisches Patentamt
## European Patent Office
## Office européen des brevets

(19)

(11) Numéro de publication: **0 117 823**
A1

(12)

# DEMANDE DE BREVET EUROPEEN

(21) Numéro de dépôt: 84400404.4

(22) Date de dépôt: 29.02.84

(51) Int. Cl.³: **C 12 N 15/00**, C 12 N 9/10, C 12 P 21/02, C 12 N 1/20 // C12R1/19, C12R1/125

(30) Priorité: 01.03.83 FR 8303320

(43) Date de publication de la demande: 05.09.84 **Bulletin 84/36**

(84) Etats contractants désignés: **AT BE CH DE GB IT LI LU NL SE**

(71) Demandeur: **Etablissement Public dit: CENTRE NATIONAL DE LA RECHERCHE SCIENTIFIQUE (CNRS), 15, Quai Anatole France, F-75007 Paris (FR)**

(72) Inventeur: **Gay, Philippe Bernard Denis, 13 bis, rue Curial, Appt. 97, F-75019 Paris (FR)**
Inventeur: **Steinmetz, Michel Henri, 100 bis, rue des Pyrénées, F-75020 Paris (FR)**
Inventeur: **Le Coq, Dominique Gilles, 9, rue Vasco de Gama, F-75015 Paris (FR)**

(74) Mandataire: **Warcoin, Jacques et al, Cabinet Régimbeau 26, avenue Kléber, F-75116 Paris (FR)**

(54) Vecteurs de clonage et d'expression du gène sacB et procédé pour la préparation de la levansaccharase.

(57) La présente invention concerne un nouveau vecteur de clonage et d'expression, comportant au moins des éléments assurant l'expression du gène sac B, et le gène sac B ainsi que l'origine de réplication d'un plasmide et préparation de la levansaccharase codée par le gène sac B.

EP 0 117 823 A1

VECTEURS DE CLONAGE ET D'EXPRESSION DU GENE SACB
ET PROCEDE POUR LA PREPARATION DE LA LEVANSACCHARASE.

La présente invention concerne des vecteurs de clonage et d'expression du gène sacB, des bactéries transformées par ces vecteurs et un procédé pour la préparation de la levansaccharase codée par le gène sacB.

La levansaccharase ou $\beta$-2,6 fructan:D glucose-1-fructosyl transférase; E.C.2.4.1.10, est une enzyme sécrétée dans le milieu de culture par Bacillus subtilis après induction par le saccharose.

Cette enzyme catalyse essentiellement la réaction suivante :

saccharose + accepteur ⟶ glucose + accepteur-fructose.

Les accepteurs possibles sont divers, par exemple l'eau (réaction d'hydrolyse) ou les levanes (réaction d'élongation des levanes).

L'un des intérêts actuel de la levansaccharase est précisément dans cette réaction d'élongation des levanes.

0117823

En effet, la réaction de transfructosylation catalysée par la levansaccharase est actuellement à l'étude afin de stocker le fructose sous forme de hauts polymères à partir du saccharose. Cette technologie pourrait, notamment, être appliquée au traitement des mélasses afin de transformer un sous produit de peu de valeur en un sucre beaucoup plus facile à valoriser.

L'un des objectifs de la présente invention est donc la mise au point d'un vecteur d'expression de la levansaccharase assurant une surproduction de l'enzyme.

C'est pourquoi la présente invention concerne des vecteurs de clonage et d'expression, caractérisés en ce qu'ils comportent au moins des éléments assurant l'expression du gène sacB, l'ADN du gène sacB, ainsi que l'origine de réplication d'un plasmide.

Ce type de vecteur plasmidique permet, par transformation de Escherichia coli, d'obtenir des extraits environ 30 fois plus actifs en levansaccharase que ceux de la souche de référence de B. subtilis.

Mais les études faites sur les vecteurs ainsi obtenus ont montré que ceux-ci pouvaient également être très utiles comme vecteurs de clonage et d'expression d'autres gènes, en particulier dans les colibacilles.

En effet, les souches de E. coli transformées par les vecteurs selon l'invention sont empoisonnées par le saccharose, il s'agit là d'un empoisonnement spécifique dont la nature exacte n'a pas encore été déterminée.

Afin d'être utilisables notamment comme vecteur de clonage d'un gène étranger, les vecteurs selon l'invention comporteront de préférence au moins un site unique de restriction situé dans une position telle que l'insertion d'une séquence d'ADN dans ce site inhibe la synthèse de la levansaccharase.

Ainsi, en utilisant ce site unique du vecteur pour cloner un gène étranger, il est aisé,après transformation de souches de E. coli par le mélange de ligation, de détecter les souches de E. coli transformées par le vecteur portant l'insertion désirée puisque dans un milieu nutritif contenant du saccharose les souches portant le plasmide sans insertion meurent.

Parmi les plasmides de l'invention, le plasmide pLS8 par exemple présente un site unique de restriction Kpn dans le gène sacB.

Les vecteurs selon l'invention peuvent comporter un site de régulation sacR (promoteur opérateur) contigu à sacB. Chez B. subtilis ce site permet la régulation de la transcription du gène sacB par les produits des gènes sacS et sacU.(Lepesant et col. 1976). Cette dernière régulation a été mise en évidence par les mutations pléiotropes du locus sacU (pap) qui affectent le niveau de synthèse de la lévansaccharase, de deux protéases exocellulaires et de l'α-amylase et altèrent diverses fonctions cellulaires telles que la synthèse des flagelles et le déclenchement de la sporulation (Kunst et col., 1976). Le mode d'action et la signification de cette régulation pléiotrope sont encore incomplètement connus.

Il est intéressant,comme cela sera démontré dans les exemples, d'utiliser un vecteur comportant un ou plusieurs des gènes de régulation mentionnés précédemment, notamment sacR ou sacU.

En effet, comme cela a été dit précédemment, chez certains mutants comportant une mutation dans le gène sacU, la synthèse de plusieurs enzymes exocellulaires, amylases, protéases, levanes, saccharases, est significativement accrue par rapport à la souche de référence.

Enfin, ces vecteurs qui assurent l'expression de la levansaccharase, protéine qui chez Bacillus est directement exportée dans le milieu de culture, constituent un point de départ intéressant pour des vecteurs d'excrétion.

Les vecteurs selon l'invention peuvent également comporter le promoteur du gène sacB.

Parmi les origines de réplication des plasmides vecteurs selon l'invention, on retiendra surtout celles des plamides susceptibles de se répliquer dans les Colibacilles, c'est-à-dire par exemple les origines de réplication de type Col El tel que celle de pBR322.

Il a été mis en évidence que le fragment portant les éléments nécessaires à l'expression de la levansaccharase était porté sur un fragment d'environ 4,1 kb autour de sacB et incorporant sacR, ce fragment étant limité par deux sites HindIII.

Ce fragment peut donc être introduit dans un plasmide tel que pBR325 (dérivé de pBR322) qui comporte un site unique HindIII,pour obtenir un plasmide pLS8 qui assure l'expression de la levansaccharase dans E. coli.

L'invention concerne également les bactéries, et notamment les souches de E. coli, transformées par les vecteurs selon l'invention comportant ou non l'insertion d'un gène codant pour une protéine autre que la levansaccharase.

Il est intéressant d'intégrer un vecteur selon l'invention dans le chromosome de B.subtilis. Cette intégration dépend de la présence dans le vecteur de séquences d'ADN homologues à celles des séquences chromosomiques. Ces dernières séquences peuvent elles mêmes être des séquences plasmidiques intégrées au préalable dans le chromosome.

L'homologie de séquence permet l'intégration par recombinaison générique mettant en jeu un seul "crossing over".

L'intégration dans le chromosome de B.subtilis d'un gène cloné en phase à la suite du promoteur de sacB permet qu'il soit mis sous la dépendance des gènes de régulation sacS et sacU avec les avantages décrits précédemment.

Enfin l'invention concerne un procédé de préparation de levansaccharase dans lequel on fait fermenter un milieu par une souche bactérienne transformée par les vecteurs selon l'invention.

Matériels et Méthodes

Souches et vecteurs de clonage

Les souches utilisées sont rassemblées dans le tableau 1.

Le plasmide pH4 est un dérivé de pHV33 décrit dans Primrose et col., 1981, obtenu par digestion partielle par HhaI. Il a perdu la fonction pC194 et le site EcoRI de la partie pBR322 de pHV33.

La nomenclature des phénotypes est la suivante :

1) $Lvs^{+}/Lvs^{-}$ indique la capacité à synthétiser la levansaccharase que l'on détecte par l'activité saccharolytique;

2) $Lvs^{c}/Lvs^{i}$ indique que la synthèse est constitutive/inductible;

3) $Lev^{+}/Lev^{-}$ indique la capacité de la levansaccharase à synthétiser des levanes sur un milieu solide approprié.

Milieux

La multiplication des phages λ Charon est supportée par la souche DP50 (supF) (Blattner, 1977) croissant sur un milieu contenant :

"Bactotryptone" (Difco)          20 g/l
"Bacto-yeast extract" (Difco)    10 g/l
NaCl                             10 g/l
$MgCl_2$                         10 mM
Acide diaminopimélique (Sigma) 100 mg/l
Thymidine (Sigma)                50 mg/l.

Pour la croissance des autres souches de E. coli on utilise un milieu LB liquide ou solide contenant :

| "Bacto-tryptone" | 10 g |
| Extrait de levure | 5 g |
| NaCl | 10 g |
| $H_2O$ | 1000 ml. |

_Bacillus subtilis_ est cultivé sur plaque "Tryptone Blood Agar Base" Difco ou sur milieu minéral MM décrit dans Anagnostopoulos et col., 1961.

Le milieu SG est un milieu minéral MM supplémenté avec 20 g/l de sucrose et 1 g/l de glucose.

Isolement et purification d'ADN

ADN de bactériophage λ :

Les lysats de phages sont concentrés par précipitation (PEG 6000 (Baker); 100 g/l, NaCl 0,5 M). Les phages sont purifiés par centrifugation sur gradient de CsCl. Les ADN sont extraits deux fois avec du phénol et le phénol residuel est extrait à l'éther. Après précipitation à l'éthanol, les ADN sont solubilisés dans TE (Tris-HCl 10 mM, pH 7,5, EDTA 1 mM).

ADN plasmidique :

Pour des isolements sur grande échelle, les souches incorporant les plasmides sont cultivées selon le processus décrit par Bolivar et col., 1977. L'amplification est obtenue par addition soit de chloramphénicol (170 mg/l) ou de spectinomycine (300 mg/l). Les ADN sont extraits selon la technique décrite par Humphreys et col., 1975. Les ADN plasmidiques sont purifiés par séparation sur $CsCl_2$ ou chromatogrphie sur hydroxyapatite,comme cela est décrit dans Colman et col., 1978. Pour des isolements sur petite échelle on utilise la procédure alcaline rapide décrite par Birnboim et Doly, 1979.

Les ADN transformants de B. subtilis sont extraits selon le procédé de Marmur, 1961.

0117823

## Analyse et clonage d'ADN

Les différentes enzymes de restriction mises en oeuvre sont disponibles dans le commerce mais peuvent également être préparées par des procédés connus, elles sont mises en oeuvre selon les processus préconisés par les fabricants ou bien par des processus équivalents.

Les électrophorèses sur plaque de gel (agarose, Sigma, réf. a6877) sont effectuées dans un tampon Tris-acétate (40 mM tris base, 20 mM acétate de sodium, 2 mM EDTA,) ajusté à pH 7,9 avec l'acide acétique. L'ADN de phage de Charon 4 soumis à une double digestion par EcoRI et BamHI ou l'ADN de pBR322 mis digestion avec HpaII sont utilisés comme référence de poids moléculaire.

## Techniques génétiques

Les souches de E. coli sont transformées selon le procédé décrit par Dagert et Erhlich, 1979. Les colonies $Cm^r$, $Tet^r$, $Amp^r$ sont sélectionnées sur des plaques LB supplémentées avec l'antibiotique correspondant, le chloramphénicol, 15 mg/l, la tétracycline, 15 mg/l et l'ampicilline 200 mg/l.

Les souches de B. subtilis sont transformées selon le procédé décrit par Anagnostopoulos et col., 1961, en utilisant la modification des milieux MG I et MG II décrite par Borenstein et col., 1969.

Les transformants prototrophiques sont sélectionnés sur plaques MM auxquelles il manque soit du tryptophane soit de la cystéine. Le phénotype Smo/Rou est déterminé sur des plaques TBAB par une technique de réplication des colonies isolées.

Le phénotype correspondant à la levansaccharase est déterminé selon le processus décrit par Lepesant et col., 1972.

Le phénotype de Lev$^+$ est identifié par la synthèse du lévane qui entoure les colonies sur des plaques SG.

Le phénotype Lvs$^c$/Lvs$^i$ est identifié sur des colonies isolées sur plaques MM supplémentées avec du glycérol au lieu de glucose.

Après 24 heures de croissance à 37°C, les plaques sont pulvérisées avec un mélange de chloramphénicol (2 mg/ml) et de saccharose (500 mg/ml) et incubées à 37°C pendant encore 90 minutes puis pulvérisées avec un réactif GOD Perid.

Le phénotype constitutif correspond à un halo gris qui entoure les colonies.

Extraits bactériens et détermination de l'activité levansaccharase

Les souches de E. coli, traitées par l'EDTA (1 mM dans Tris-HCl 100 mM, pH 7,5), sont sédimentées et remises en suspension dans un tampon phosphate de potassium 50 mM, pH 6,0. On ajoute 1 mg/ml de lysozyme et les cellules sont mises en incubation à 0°C pendant 30 minutes. La lyse est achevée par refroidissement et décongélation. Les extraits sont traités par de la DNase, 50 µg/ml, de façon à éviter une viscosité excessive.

La levansaccharase est dosée par la méthode décrite par Chambert et col., 1974. On utilise du saccharose ou du glucose marqué au carbone 14 de façon uniforme, qui est fourni par Amersham corp.

0117823

Ces sucres sont purifiés par chromatographie sur papier avant leur utilisation.

La transfructosylation sur glucose (réaction d'échange) est essayée sur le mélange suivant :

Glucose C$^{14}$          200 mM (0,1 μCi/μM)

Sucrose                   100 mM

Tampon phosphate de potassium      50 mM

pH 6,0.

La transfructosylation sur des levanes est mise en évidence dans un mélange comprenant :

Sucrose C$^{14}$      240 mM

Levanes purifiés (poids moléculaire 10 000)      10 mg/ml

Tampon phosphate de potassium      50 mM

pH 6,0.

En l'absence de levane à titre d'accepteur, l'incubation de levansaccharase dans ce mélange conduit uniquement à l'hydrolyse du saccharose.

Dans tous les cas des aliquots des mélanges de réaction sont soumis à une chromatographie sur papier (papier Schleicher et Schüll 2043a, solvant : n-butanol/ acide acétique/eau - 4/1/1, v/v/v).

Les taches radioactives sont localisées par autoradiographie. La radiographie de chacune des taches est estimée directement à partir d'un papier immergé dans un fluide de scintillation.

L'activité de la levansaccharase synthétisée par les souches de E. coli peut être également révélée sur milieu solide par un test adapté du test mis au point pour B. subtilis par Lepesant et col., 1972. Après 24 heures de croissance à 37°C sur milieu solide M63 + glycérol + CM (25 μg/ml) les colonies sont :

1) recouvertes par pulvérisation avec un mélange de lysozyme (10 mg/ml) et d'EDTA ($10^{-3}$ M) et incubées 30 minutes à 37°C ;

2) recouvertes avec une solution de saccharose (1,5 M) et incubées 60 minutes à 37°C ;

3) recouvertes avec le réactif du glucose GOD-Perid (Boehringer).

Un halo vert autour des colonies révèle le glucose produit à partir du saccharose par la levansaccharase.

EXEMPLE 1

Isolement d'un clone portant le gène sacB
d'une banque de phages λ charon 4A

La construction d'une banque d'ADN de
B. subtilis provenant de la souche 168 M (trpC, smo)
clonés dans les sites EcoRI du phage λ charon 4 a
été décrite dans l'article de Ferrari et col., 1981.

Lorsque cette banque a été testée pour son
activité de transformation sur des souches de B. subtilis
compétentes, 70 % des marqueurs testés ont été détectés
sur un échantillon de 1710 plaques.

Le même échantillon a été testé pour détecter
la présence de clones qui portent le gène de structure
codant pour l'exoenzyme levansaccharose.

La transformation des allèles sacB dans le type
sauvage ne permet pas une sélection directe des
recombinants. Toutefois, les types sauvages Lev$^+$
peuvent être distingués des Lev$^-$ sur milieu SG
car ils sont entourés de levane. De façon à identifier
les colonies Lev$^+$ dans les populations transformées,
les transformants sont sélectionnés par congression
avec un marqueur d'auxotrophie non lié.

Une culture compétente de souche QB1058
(sacB197, trpC2, sacS2) est exposée à une quantité
sous saturante d'ADN de la souche PG790 (sacB197, sacS2)
et étalée sur un milieu SG pour obtenir moins de
5000 colonies trp$^+$ par plaque.

Les ADN provenant de chacun des 19 sous-ensembles
d'environ 90 plaques de phages λ recombinants sont
disposés sur des cellules compétentes.

Parmi les colonies Trp$^+$ ainsi sélectionnées, les colonies Lev$^+$ sont trouvées sur deux taches ; ceci indique que certains clones recombinants portant l'allèle sacB sont présents dans deux sous-ensembles.

Les lysats bruts sont préparés à partir de chaque plaque d'un sous-ensemble et sont utilisés comme source d'ADN dans les expériences de transformation décrites.

Ceci permet l'identification et l'isolement d'une plaque λ LSC66, dont l'ADN transforme l'allèle sacB197. Le clone est purifié deux fois et cultivé pour en obtenir des quantités importantes.

EXEMPLE 2

Le produit de digestion de l'ADN de λ LSC66 par HindIII est mis en ligation dans le site HindIII du plasmide pH4.

Les plasmides obtenus sont utilisés pour transformer une souche de E. coli. La sélection des transformants Amp$^r$, Tet$^s$ permet d'isoler un clone incorporant le plasmide pLS5.

Ce plasmide n'est pas capable d'assurer l'activité levansaccharase dans une souche hôte de E. coli. Afin de construire un plasmide plus complet, on insère dans le site unique EcoRI de pLS5 un fragment de restriction EcoRI de l'ADN de λ LSC66.

Le plasmide recombinant pLS7 est identifié en analysant par restriction l'ADN des souches de E. coli transformées Amp$^r$.

L'insert de 5,5 kb de ce plasmide contient les 4,1 kb de l'extrémité à gauche de λ LSC66, une partie de ce matériel est en double.

Les transformants E. coli pLS7 synthétisent la levansaccharase. Toutefois, les colonies Lvs$^+$ de E. coli tendent à ségréger des colonies Lvs$^-$ avec un taux assez élevé. Ces colonies Lvs$^-$ possèdent un plasmide similaire à pLS5.

## EXEMPLE 3

Afin d'obtenir un plasmide présentant une stabilité améliorée, on insère un fragment de restriction HindIII de λ LSC66 dans pBR325. Le mélange de digestion de l'ADN de λ LSC66 par HindIII est mis en ligation dans le site HindIII du plasmide pBR325 et le mélange de ligation obtenu est utilisé pour transformer la souche de E. coli HVC45. L'étude des transformants permet l'isolation de transformants portant le plasmide pLS8 dans lequel le gène Tet de pBR325 a été inactivé.

## EXEMPLE 4
### Identification de la levansaccharase dans
### une souche de E. coli incorporant pLS8

Un extrait brut de la souche HVC45 portant le plasmide pLS8 est obtenu comme cela a été décrit précédemment.

L'activité fructosyl transférase de la levansaccharase synthétisée par cette souche est caractérisée par trois réactions différentes :

1) la réaction d'échange (transfert fructosyl du saccharose au glucose radioactif ;

2) hydrolyse du saccharose ;

3) transfert fructosyl à un accepteur levane.

Comme aucune réaction d'échange et aucune synthèse de levane ne peut être mise en évidence dans les souches de E. coli incorporant des vecteurs qui ne contiennent pas l'allèle sacB, ces expériences démontrent l'expression du gène sacB.

L'activité spécifique de l'enzyme est d'environ 0,8 unité par mg de protéines. Ceci correspond à un taux de synthèse d'environ $10^{-11}$ unité d'enzyme par cellule par minute. Ce taux évidemment dépend du nombre de copies du gène sacB dans la cellule.

pLS8 qui dérive du plasmide pBR325 présente un mode de réplication Col E1.

Clewell et col., 1972, ont montré que des plasmides ColE1 étaient présents dans E. coli au taux d'environ 24 copies par cellule. Les études sur pLS8 sans amplification permettent d'estimer le nombre des plasmides au même ordre de grandeur.

Dans B. subtilis le taux de levansaccharase mesuré sur la souche de référence 168M est de l'ordre de $10^{-12}$ unité/cellule/minute.

En milieu liquide, LB + CM, la vitesse de croissance de la souche comportant le plasmide pLS8 est semblable à celle de la souche parente portant pBR325. L'addition de saccharose a un effet toxique sur la première, mais n'affecte pas la deuxième.

L'addition de saccharose (150 mM) est suivie 40 à 60 minutes plus tard d'une lyse rapide mise en évidence par la chute de densité optique et confirmée par observation microscopique. Cette lyse affecte en 30 minutes 40 à 70 % de la population, puis se poursuit à une vitesse beaucoup plus lente. Le comptage des bactéries viables par étalement sur milieu solide LB + CM montre que c'est en fait la quasi-totalité de la population qui est empoisonnée de manière irréversible. L'addition de saccharose à des concentrations plus faibles (60 mM et 100 mM) donne lieu à des empoisonnements plus lents.

Ni la localisation cellulaire de cette intoxication, ni son mécanisme ne sont connus avec certitude.

Les études effectuées sur des mutants comportant le plasmide pLS8 muni d'une insertion dans le segment correspondant au gène sacB ont montré que ceux-ci résistent à l'effet toxique du saccharose.

Ceci permet donc d'envisager l'utilisation des vecteurs selon la présente invention comme vecteurs de clonage et d'expression pour des gènes autres que sacB en assurant une sélection aisée des mutants désirés.

## TABLEAU 1

### LISTE DES SOUCHES

| Souche | Génotype | Phénotype |
|---|---|---|
| **B. subtilis** | | |
| 168M | trpC2 smo | Trp⁻ Smo |
| QB1058 | sacA321 sacB197 sacS32 trpC2 smo | Trp⁻ Lev⁻ Smo |
| PG790 | sacA321 sacB197 sacS32 smo | Lev⁻ Smo |
| | | |
| **E. coli** | | |
| DP50 supF | F⁻ dapD8 lacY Δ{gal uvrB} ΔthyA malAʳ hdsS suII suIII | |
| HVC45 | thrA1 leu6 thi1 lacY1 supE44 hsdR strR tonA1 | Ampˢ Cmˢ Tetˢ |

## REFERENCES BIBLIOGRAPHIQUES

Anagnostopoulos C. et Spizizen J., 1961. Requirements for transformation in <u>Bacillus subtilis</u>. J. Bacteriol. 81:741-746.

Birnboim H.C. et Doly J., 1979. A rapid alkaline procedure for screening recombinant plasmid DNA. Nucl. Acids Res. <u>7</u>:1513-1523.

Blattner F.R., Williams B.G., Blechl A.E., Thompson K.D., Faber H.E., Furlong L.A., Grunwald C.J., Kiefer D.O., Moore D.D., Schumm J.N., Sheldon E.L. et Smithies O., 1977. Charon phages, safer derivatives of bacteriophage lambda for DNA cloning. Science <u>196</u>:161-169.

Bolivar F., Rodriguez R.L., Betlach M.C. et Boyer H.W., 1977. Construction and characterization of new cloning vehicles: I. Ampicillin resistant derivatives of the plasmid pMB9. Gene <u>2</u>:75-93.

Borenstein S. et Ephrati-Elizur E., 1969. Spontaneous release of DNA in sequential genetic order by <u>Bacillus</u> <u>subtilis</u>. J. Mol. Biol. <u>45</u>:137-152.

Chambert R., Gonzy-Treboul G. et Dedonder R., 1974. Kinetic studies of <u>Bacillus subtilis</u> levansucrase. Eur. J. Biochem. <u>41</u>:285-300.

Clewell D.B. et Helinski D.R., 1972. Effect of growth conditions on the formation of the relaxation complex of supercoiled Col EI deoxyribonucleic acid and protein in <u>Escherichia coli</u>. J. Bacteriol. <u>110</u>:1135-1146.

0117823

Colman A., Byers M.J., Primrose S.B. et Lyons A., 1978.
Rapid purification of plasmid DNA by hydroxyapatite
chromatography. Eur. J. Biochem. 91:303-310.

Dagert M. et Ehrlich S.D., 1979. Prolonged incubation in
calcium chloride improves the competence of the
Escherichia coli cells. Gene 6:23-28.

Ferrari E., Henner D.J. et Hoch J.A., 1981. Isolation
of Bacillus subtilis genes from a Charon 4A library.
J. Bacteriol. 146:430-432.

Humphreys G.O., Willshaw G.A. et Anderson E.S., 1975.
A simple method for the preparation of large quantities
of pure plasmid DNA. Biochem. Biophys. Acta 383:457-463.

Kunst F., Pascal M. Lepesant-Kejzlarova J., Lepesant J.A.
Billault A. et Dedonder. D. 1974. Pleiotropic mutations
affecting sporulation conditions and the syntheses of
extracellular enzymes in Bacillus subtilis 168. Biochimie
56:1481-1489.

Lepesant J.A., Kunst F., Lepesant-Kejzlarova J. et
Dedonder R., 1972. Chromosomal location of mutations
affecting sucrose metabolism in Bacillus subtilis Marburg.
Molec. Gen. Genet. 118:135-160.

Lepesant J.A., Kunst F., Pascal M.,Lepesant-Kejzlarova J.,
Steinmetz M. et Dedonder R., 1976. Specific and pleiotropic
regulatory mechanisms in the sucrose system of Bacillus
subtilis 168, p. 58-69. In D. Schlessinger (ed.),
Microbiology - 1976. American Society for Microbiology,
Washington D.C.

Marmur J., 1961. A procedure for the isolation of
deoxyribonucleic acid from microorganisms. J. Mol. Biol.
3:208-218.

Primrose S.B. et Ehrlich S.D., 1981. Isolation of plasmid deletion mutants and study of their instability. Plasmid 6:193-201.

REVENDICATIONS

1) Vecteur de clonage et d'expression, caractérisé en ce qu'il comporte au moins des éléments assurant l'expression du gène sacB, et le gène sacB ainsi que l'origine de réplication d'un plasmide.

2) Vecteur selon la revendication 1, caractérisé en ce qu'il comporte le promoteur de sacB.

3) Vecteur selon l'une des revendications 1 et 2, caractérisé en ce qu'il comporte le gène sacR.

4) Vecteur selon l'une des revendications 1 à 3, caractérisé en ce qu'il comporte au moins l'un des gènes de régulation de sacB.

5) Vecteur selon la revendication 4, caractérisé en ce qu'il comporte le gène de régulation sacU.

6) Vecteur selon l'une des revendications 1 à 5, caractérisé en ce qu'il comporte l'origine de réplication de Col El.

7) Vecteur selon l'une des revendications 1 à 6, caractérisé en ce qu'il comporte l'ADN du plasmide pBR325 et une insertion de 4,1 kb limitée par deux sites HindIII portant le gène sacR et le gène sacB situés dans le site unique de HindIII de pBR325.

8) Vecteur selon l'une des revendications 1 à 7, caractérisé en ce qu'il comporte,en outre, au moins un site unique de restriction situé à un endroit tel que l'insertion d'une séquence d' ADN dans ce site inhibe la synthèse de levansaccharase.

9) Vecteur selon l'une des revendications 1 à 8, caractérisé en ce qu'il comporte la séquence d'ADN codant pour une protéine déterminée autre que la levansaccharase sous la dépendance des éléments assurant l'expression du gène sacB.

22                    0117823

10) Bactérie transformée par un vecteur selon l'une des revendications 1 à 8.

11) Bactérie transformée par un vecteur selon la revendication 9.

12) Bactérie selon l'une des revendications 10 et 11, caractérisée en ce qu'il s'agit d'une souche d'<u>Escherichia coli</u>.

13) Bactéries selon l'une des revendications 10 et 11, caractérisées en ce qu'il s'agit de souche de <u>B.subtilis</u> transformée par intégration des vecteurs.

14) Procédé de préparation de levansaccharase caractérisé en ce qu'on fait fermenter un milieu de culture par une bactérie selon l'une des revendications 10 à 13.

15) Procédé de préparation d'une protéine déterminée, caractérisé en ce qu'on fait fermenter un milieu de culture par une bactérie selon l'une des revendications 11, 12 et 13.

16) Levansaccharase obtenue par la mise en oeuvre du procédé selon la revendication 14.

Office européen
des brevets

**RAPPORT DE RECHERCHE EUROPEENNE**

0117823

Numéro de la demande

EP 84 40 0404

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl. 3) |
|---|---|---|---|
| X | METHODS IN ENZYMOLOGY, vol. 8, 1966, pages 500-505 R. DEDONDER: "Levansucrase from Bacillus subtilis" * En entier * --- | 16 | C 12 N 15/00 C 12 N 9/10 C 12 P 21/02 C 12 N 1/20 // C 12 R 1/19 C 12 R 1/125 |
| P,X | JOURNAL OF BACTERIOLOGY, vol. 153, no. 3, 4 mars 1983, pages 1424-1431, American Society for Microbiology P. GAY et al.: "Cloning structural gene sacB, which codes for exoenzyme levansucrase of Bacillus subtilis: expression of the gene in Escherichia coli" * En entier * --- | 1-8,10 ,12,14 -16 | |
| P,X | CHEMICAL ABSTRACTS, vol. 99, no. 17, 24 octobre 1983, page 144, no. 134583v, Columbus, Ohio, US M. STEINMETZ et al.: "Genetic analysis of sacB, the structural gene of a secreted enzyme, levansucrase, of Bacillus subtilis Marburg" & MGG, MOL. GENET. 1983, 191(1), 138-44 * Résumé * --- | 1-8,10 ,12-16 | DOMAINES TECHNIQUES RECHERCHES (Int. Cl. 3) C 12 N C 12 P C 12 R |
| A | CHEMICAL ABSTRACTS, vol. 98, no. 21, 23 mai 1983, page 201, no. 174119j, Columbus, Ohio, US E. FERRARI et al.: "Isolation of Bacillus subtilis genes from Charon libraries" & MICROBIOLOGY (WASHINGTON, D.C.), 1982, 3-4 * Résumé * --- -/- | 1 | |

Le présent rapport de recherche a été établi pour toutes les revendications

| Lieu de la recherche LA HAYE | Date d'achèvement de la recherche 22-05-1984 | Examinateur DESCAMPS J.A. |
|---|---|---|

# RAPPORT DE RECHERCHE EUROPEENNE

0117823
Numéro de la demande

Office européen
des brevets

EP  84 40 0404

| | DOCUMENTS CONSIDERES COMME PERTINENTS | | Page  2 |
|---|---|---|---|
| **Catégorie** | **Citation du document avec indication, en cas de besoin, des parties pertinentes** | **Revendication concernée** | **CLASSEMENT DE LA DEMANDE (Int. Cl. 3)** |
| A | MOLEC. GEN. GENET., vol. 128, 1974, pages 213-221, Springer-Verlag J.A. LEPESANT et al.: "Identification  of the structural gene of levansucrase in Bacillus subtilis Marburg" * <br><br> --- <br><br> ----- | | |
| | | | **DOMAINES TECHNIQUES RECHERCHES (Int. Cl. 3)** |
| | Le présent rapport de recherche a été établi pour toutes les revendications | | |

| Lieu de la recherche | Date d'achèvement de la recherche | Examinateur |
|---|---|---|
| LA HAYE | 22-05-1984 | DESCAMPS J.A. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

 

& : membre de la même famille, document correspondant

OEB Form 1503. 03.82